# EUROPEAN PATENT SPECIFICATION

(11) **EP 4 142 832 B1**
(45) Date of publication and mention of the grant of the patent: **31.12.2025**
(21) Application number: 21722304.9
(22) Date of filing: 30.04.2021
(51) Int. Cl.: A61M 5/178, A61M 5/31

(54) **SYRINGE FOR IMPROVED INJECTION AND ASPIRATION**
SPRITZE FÜR VERBESSERTE INJEKTION UND ASPIRATION
SERINGUE POUR INJECTION ET ASPIRATION AMÉLIORÉES

(30) Priority: 30.04.2020 EP 20172257
(43) Date of publication of application: 08.03.2023
(73) Proprietor: Galderma Holding SA, 6300 Zug (CH)
(72) Inventor: TYRSING, Oliver, 756 44 Uppsala (SE); ULLSTEN, Oscar, 753 30 Uppsala (SE)
(74) Representative: Impact Intellectual Property LLP
(86) International application number: PCT/IB2021/053601
(87) International publication number: WO 2021/220228

(56) References cited:
- KR-B1- 101 900 427
- US-A1- 2009 137 966
- US-A1- 2012 220 948
- US-A1- 2018 243 513

## Description

### Field of the invention

The present invention relates to a syringe for injection and aspiration, more in particular a syringe comprising a tube arranged to hold a liquid material, a plunger rod having a plunger movably arranged within the tube, and a plunger rod head attached to the plunger rod opposite from the plunger, the plunger rod head having a first surface and a second surface opposite to the first surface, wherein a first non-slip surface part is provided on at least a part of the first surface.

### Background art

International publication WO2017/176476 discloses a device for aspiration and injection, allowing for ergonomic, safe, and precise aspiration of a target site and ejection of a medicament to the target site. The device can include a syringe barrel, a flange extender that can be coupled to the barrel, and a plunger that can be engaged by a hand and/or one or more finger of a user to perform aspiration and injection at a target site.

### Summary of the invention

The present invention seeks to provide a device for injection and aspiration, based on a syringe that comprises a combination of ergonomic features that improves user performance.

According to the exemplary embodiments thereof, a syringe as defined above is provided, wherein a second non-slip surface part is provided on at least a part of the second surface, and the syringe further comprises a finger grip component, comprising a first portion extending away from a longitudinal axis of the syringe and a second portion extending away from the longitudinal axis of the syringe. The finger grip component is a one-piece body attached to the tube, comprising an injection finger support surface with a third non-slip surface part positioned in the first portion and arched in a direction perpendicular to the longitudinal axis with a first curvature for co-operative association with the first surface of the plunger rod head, and an aspiration finger support surface with a fourth non-slip surface part positioned in the second portion and arched in a direction perpendicular to the longitudinal axis with a second curvature for co-operative association with the second surface of the plunger rod head.

The injection finger support surface is thus provided with an ergonomic curvature for co-operative association with the first surface of the plunger rod head, and an aspiration finger support surface with an ergonomic curvature for co-operative association with the second surface of the plunger rod head. By combination of the ergonomic curved surfaces and/or non-slip surface parts provided on the plunger rod head, injection finger support surface and aspiration finger support surface, this provides improved user handling and comfort of the syringe. This provides a syringe for improved aspiration and injection, comprising a combination of ergonomic features that improves the user performance, allowing a steadier grasp on the syringe and easy performance of both aspiration and injection using one hand.

### Short description of drawings

Fig 1. shows a side view of a syringe for improved aspiration and injection according to an exemplary embodiment;
Fig 2 is a side view of a finger grip component according to an exemplary embodiment;
Fig. 3 shows a perspective view of a finger grip component of Fig. 2; and
Fig. 4 shows a side view of a plunger rod head according to an exemplary embodiment.

### Description of embodiments

Various syringes are known in the art for use on a human or animal subject. Many syringes are used for injection purposes, for example, for delivering drugs such as anesthetic for surgical procedures or for delivering aesthetic solutions, e.g. in skin regions. Similarly, syringes are also used for aspiration purposes, for example, withdrawing blood from a subject for further medical analysis. In certain applications, syringes are used for both injection and aspiration purposes, that is, syringes that facilitate both delivery and withdrawal of a material, where the material is typically a liquid. Also, typically, syringes for delivering aesthetic solutions use both methods, first for checking the needle tip is not in a blood vessel or artery, and if no blood is drawn, the aesthetic solution is injected. As such, the variety of syringes known in the art can be found in many sizes and shapes, where a syringe can be tailor-made for a certain application.

A conventional syringe generally comprises a needle, a tube, and a plunger, where the plunger is movably arranged within the tube to inject or aspirate the contents therein. In general, it may be desirable for a user of these conventional syringes to use manual actuation by a single hand of the user. That is, a user may prefer to use their thumb and fingers to manually actuate the plunger within the tube. The advantages of manual actuation is that the user can easily switch the syringe of choice, they are easy to operate, and no external power source is required. As such, many conventional syringes known in the art include ergonomic features for improved user performance, for example, finger grip components or thumb plates that give additional grip or control of the syringe to the user.

Nevertheless, these conventional syringes for manual actuation still have their drawbacks. In certain applications, for example, aesthetic treatments that require injecting Hyaluronic acid- (HA) based dermal fillers, thousands of injections or aspirations may be required for the patient. This requires long periods of user concentration, where the user must maintain good grip and control of the syringe to safely carry out the aesthetic treatment. This leads to user exhaustion, and thereby the disadvantage of manual actuation is that the user performance is severely limited. Although conventional syringes include ergonomic features that may improve a certain element of the user performance, there is a need in the art for a syringe that improves several elements of the user performance, and overcome this disadvantage. This would allow the user to perform a large number of injection and aspiration actions over a longer period of time, in a safe and ergonomic manner.

The exemplary embodiments provides a syringe for improved aspiration and injection, comprising a combination of ergonomic features that improves the user performance.

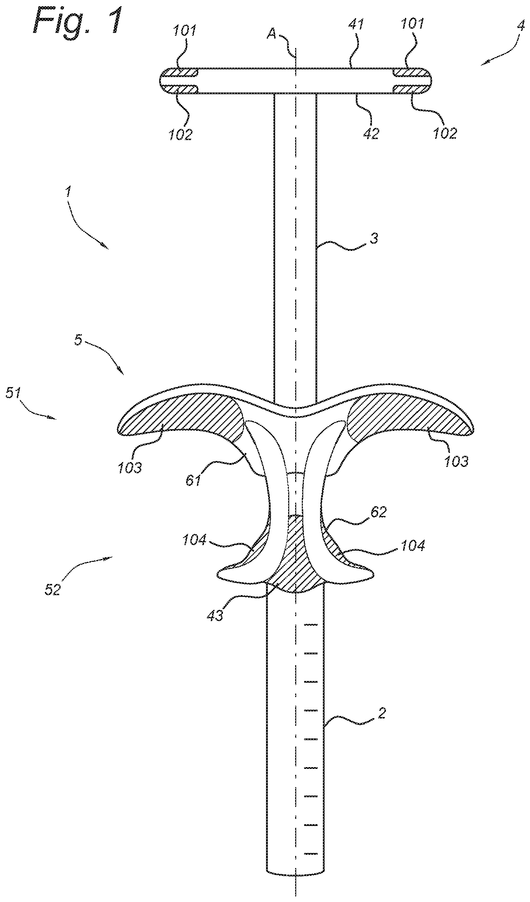

Fig. 1 shows a perspective view of a syringe 1 for aspiration and injection according to an exemplary embodiment. The syringe 1 comprises a tube 2, arranged to hold a liquid material. The liquid material in the tube 2 may be externally added by a user for injection processes, or the liquid material may be withdrawn into the tube 2 by a user in aspiration processes. The tube 2 may comprise of a transparent solid material, for example, glass or polycarbonate, with a measuring scale on the outer surface of the tube 2, allowing the user to measure the amount of liquid material to be injected and/or aspirated.

In the embodiment shown in Fig. 1, the syringe 1 further comprises a plunger rod 3, movably arranged within the tube 2, along the direction parallel to the longitudinal axis A of the tube 2. The plunger rod 3 comprises a plunger 43. The tube 2 further comprises an open end, where the plunger rod 3 may be inserted into or withdrawn from. The plunger 43 of the plunger rod 3 is arranged to sealingly engage against the inner surface of the tube 2, creating a tight seal therefrom. This allows no escape of the liquid material in the tube 2 through the tight seal between the plunger 43 and the inner surface of the tube 2, thereby sealing the liquid material within the tube 2. Although a resistive force may be present at the tight seal between the plunger 43 and the inner surface of the tube 2, the plunger rod 3 can freely move within the tube 2 without excessive forces.

A plunger rod head 4 (or thumb plate) is attached to the end of the plunger rod 3, opposite to the plunger of the plunger rod 3, where the plunger rod head 4 can be used to control and move the plunger rod 3 within the tube 2. The plunger rod head 4 comprises a first surface 41, and a second surface 42, where the second surface 42 is opposite to the first surface 41, wherein a first non-slip surface part 101 is provided on at least a part of the first surface 41, and a second non-slip surface part 102 is provided on at least part of the second surface 42. In an exemplary embodiment, the entire first surface 41 is a non-slip surface. For example, the first surface 41 and the second surface 42 can be provided with a layer of the first non-slip surface part 101 and the second non-slip surface part 102, respectively. Alternatively, the entire first surface 41 and the entire second surface 42 can be provided with a layer of non-slip material to form the first non-slip surface part 101 and the second non-slip surface part 102.

In an embodiment, the first non-slip surface part 101 and second non-slip surface part 102 comprise a first material, which is softer than a second material of the associated first surface 41 and second surface 42. In further embodiments, the first material comprises a thermoplastic elastomer, and the second material comprises a polypropylene material.

The first (e.g. thermoplastic elastomer) material comprises a softer surface in comparison to the surface of the second (e.g. polypropylene) material, allowing for improved comfort when contact is made with the user's hand and/or finger(s). The first non-slip surface part 101 and second non-slip surface part 102 may further comprise a surface with enhanced grip in comparison to the surface of the polypropylene material, e.g. in the form of a textured surface, allowing improved user handling when contact is made with the user's hands and/or finger(s).

This feature of a first non-slip surface part 101 provided on at least a part of the first surface 41, and the second non-slip surface part 102 provided on at least a part of the second surface 42, where the first non-slip surface part 101 and second non-slip surface part 102 comprise a soft grip thermoplastic elastomer material, provides improved ergonomic user handling and comfort.

In the embodiment shown in Fig. 1, the syringe 1 further comprises a finger grip component 5, wherein the finger grip comprises a first portion 51 extending away from a longitudinal axis A of the syringe 1, and a second portion 52 extending away from the longitudinal axis A of the syringe 1. The finger grip component 5 allows the user to achieve a steady grasp on the syringe 1, where the finger grip component 5 works in co-operative association with the plunger rod head 4 for aspiration and injection. In this embodiment, the finger grip component 5 is a one-piece body attached or attachable to the tube 2, comprising an injection finger support surface 61, positioned in the first portion 51 and arched in a direction perpendicular to the longitudinal axis A with a first curvature for co-operative association with the first surface 41 of the plunger rod head 4. The finger grip component 5 further comprises an aspiration finger support surface 62, positioned in the second portion 52 and arched in a direction perpendicular to the longitudinal axis A with a second curvature for co-operative association with the second surface 42 of the plunger rod head 4. The structure of the finger grip component 5 comprises ergonomic features that allows the user to achieve a steadier grasp on the syringe 1.

In the embodiment shown in the side view of Fig. 1, the injection finger support surface 61, positioned in the first section 51, extends away from the longitudinal axis A, and is arched in a direction perpendicular to the longitudinal axis A. The injection finger support surface 61 comprises a first curvature of a smooth, convex nature, curving inwardly and away from the central part of the finger grip component. The convex curvature of the injection finger support surface 61 allows the user to support at least a part of their finger(s) or thumb upon, and in co-operative association with the first surface 41 of the plunger rod head 4, to easily perform injection using only one hand (i.e., single-handedly).

For example, the user may support a part of their index and/or middle finger on the convex curvature of the injection finger support surface 61, and for co-operative association, support a part of their thumb, of the same hand, on the first surface 41 of the plunger rod head 4. This configuration can allow the user to use their thumb and force the plunger rod head 4 towards the tube 2, thereby moving the plunger rod 3 into the tube 2 to perform injection of the liquid material within the tube 2, whilst maintaining a steady grasp of the syringe 1.

Similarly, in view of Fig. 1, the aspiration finger support surface 62 also extends from the longitudinal axis A, and is arched in a direction perpendicular to the longitudinal axis A. The aspiration finger support surface 62 comprises a second curvature of a smooth, concave nature, curving outwardly and into the central part of the finger grip component. The concave curvature of the aspiration finger support surface 62 allows the user to support at least a part of their finger(s) or thumb upon, and in co-operative association with the second surface 42 of the plunger rod head 4, to easily perform aspiration using one hand.

For example, the user may support part of their index and/or middle finger on the concave curvature of the aspiration finger support surface 62, and for co-operative association, support part of their thumb, of the same hand, on the second surface 42 of the plunger rod head 4. This configuration can allow the user to use their thumb and force the plunger rod head 4 away from the tube 2, thereby withdrawing the plunger rod 3 out of the tube 2 to perform the aspiration process, whilst maintaining a steady grasp of the syringe 1.

As such, the features of the injection finger support surface 61 and the aspiration finger support surface 62 provides curved, ergonomic surfaces that allows the user to easily support at least one of their fingers and/or thumbs to perform aspiration and/or injection, and thereby allowing improved user handling. It is noted that the examples provided above for aspiration or injection are non-limiting, and a combination of other hand/finger grip positions can be used to perform the injection or aspiration.

In the exemplary embodiment shown in Fig. 1, the injection finger support surface 61 and/or the aspiration finger support surface 62 have a dimension perpendicular to the longitudinal axis (A) of at least about 1 cm, e.g. at least about 2 cm.

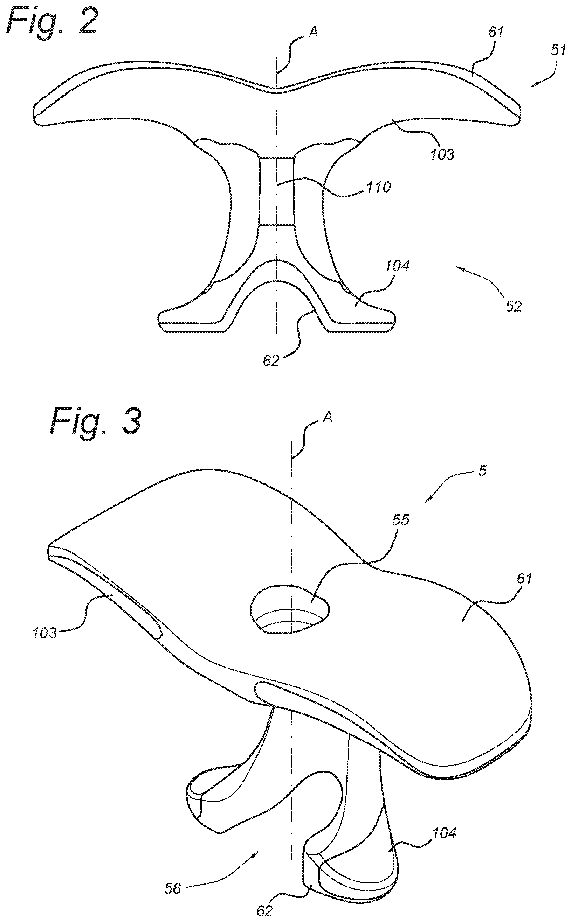

The dimensions of the injection finger support surface 61 and/or the aspiration finger support surface 62 provides sufficient surface area for good support of at least one finger. In a further exemplary embodiment, a dimension perpendicular to the longitudinal axis A of the injection finger support surface 61 is larger than a dimension perpendicular to the longitudinal axis A of the aspiration finger support surface 62. The surface area of the injection finger support surface 61 can be larger than the surface area of the aspiration finger support surface 62, such that the injection finger support surface 61 provides improved support of at least one of the fingers and/or thumbs. In an exemplary embodiment, an inner diameter of the finger grip component 5 in which the tube 2 is arranged is about 8 mm, the injection finger support surface 61 extends 18 mm from the inner diameter, and the aspiration finger support surface 62 extends 6.5 mm from the inner diameter. In the embodiment shown in Fig. 2, the total width of the first portion 51 is then 44 mm, and the total width of the second portion 55 is then 21 mm. This provides a comfortable grip for various finger positions both in an aspiration and in an injection mode of operation.

In the embodiment shown in Fig 1, the injection finger support surface 61 is provided with a third non-slip surface part 103, and the aspiration finger support surface 62 is provided with a fourth non-slip surface part 104. The third non-slip surface part 103 and fourth non-slip surface part 104 e.g. comprise the first material, and the first portion 51 and second portion 52 comprise the second material. The first material and second material are the thermoplastic elastomer and polypropylene material, respectively, similar to the embodiments of the first and second non-slip surface parts 101, 102 as described above.

In at least one exemplary embodiment, a device includes an injection finger support surface (61) with a third non-slip surface part (103) positioned in the first portion (51) and arched in a direction perpendicular to the longitudinal axis (A) with a first curvature. The non-slip surface part (103) faces away from for co-operative association with the first surface (41) of the plunger rod head (4). The device further includes an aspiration finger support surface (62) with a fourth non-slip surface part (104) positioned in the second portion (52) and arched in a direction perpendicular to the longitudinal axis (A) with a second curvature. The fourth non-slip surface part (104) faces toward for co-operative association with the second surface (42) of the plunger rod head (4), wherein the second surface (42) faces the finger grip component (5).

At least a part of the injection finger support surface 61 and at least a part of aspiration finger support surface 62 are thereby also provided with the thermoplastic elastomer material, allowing for more grip and improved comfort for the user when performing injection and/or aspiration.

In more general wording, the present embodiments relate to a syringe 1 for aspiration and injection, comprising a tube 2 arrange to hold a liquid material, a plunger rod 3 having a plunger 43 movably arranged within the tube 2, and a plunger rod head 4 attached to the plunger rod 3 opposite from the plunger 43, the plunger rod head having a first surface 41, and a second surface 42 opposite to the first surface 41, wherein a first non-slip surface part 101 is provided on at least a part of the first surface 41 and a second non-slip surface part 102 provided on at least a part of the second surface 42. The syringe 1 further comprises a finger grip component 5, comprising a first portion 51 extending away from longitudinal axis A of the syringe 1, and a second portion 52 extending away from the longitudinal axis A of the syringe 1, wherein the finger grip component 5 is a one-piece body attached to the tube 2, comprising an injection finger support surface 61 with a third non-slip surface part 103 positioned in the first portion 51 and arched in a direction perpendicular to the longitudinal axis A with a first curvature for co-operative association with the first surface 41 of the plunger rod head 4, and an aspiration finger support surface 62 with a fourth non-slip surface parts 104, positioned in the second portion 52 and arched in a direction perpendicular to the longitudinal axis A with a second curvature for co-operative association with the second surface 42 of the plunger rod head 4.

In an exemplary embodiment as shown in the side view of Fig. 2, the third non-slip surface part 103 and the forth non-slip surface part 102 are connected by an intermediate non-slip surface part 110, thereby forming a single, connected first non-slip surface layer. This eases manufacturing and the costs therefrom, as the single, connected first non-slip surface layer 110 is easier to apply to the finger grip component 5.

In an exemplary embodiment as shown in the perspective view of Fig. 3, the finger grip component comprises an opening 55 aligned with the longitudinal axis A. The diameter of the opening 55 is larger than the diameter of the cylindrical body of the plunger rod 3, allowing the plunger rod 3 to easily insert into or withdraw out of the opening 55.

The finger grip component 5 further comprises a partial circumferential aperture 56 parallel to the longitudinal axis A. In a further embodiment, the aperture 56 and intermediate non-slip surface part 110 are on opposite circumferential sides. In this exemplary embodiment, the finger grip component 5 is a separate component of the syringe 1, wherein the aperture 56 is arranged to facilitate the attachment of the finger grip component 5 to the tube 2. Further, the aperture 56 also allows local viewing of the tube and the contents therein, where the user can, for example, fill the tube with the liquid material, and, in co-operative association with a measuring scale on the outer side surface of the tube 2, view the filling of the tube 2 up to the desired amount.

The aperture 56 may comprise a width that is slightly less than the diameter of the tube 2, whereby a snap-fit attachment of the finger grip component 5 to the tube 2 can be allowed. Alternatively, the aperture 56 may comprise a slot, and by including flanges on the tube 2 that extend away from the dimension perpendicular to the perpendicular axis A, the slot on the aperture 56 can engage with the flanges on the tube 2, and a slide-on arrangement can be allowed. The attachment of the finger grip component 5 to the tube 2 can also allow for an agile grip around the tube 2, where the agile grip prevents unintentional movement or slipping of the finger grip component on the tube 2 during injection or aspiration.

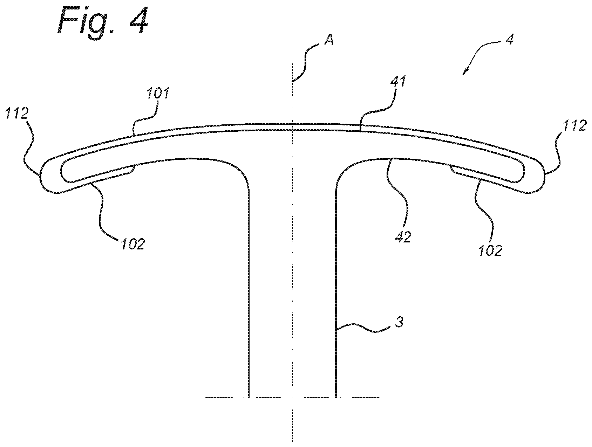

In the exemplary embodiment shown in Fig. 4, the second surface 42 of the plunger rod head 4 is arched in a direction perpendicular to the longitudinal axis A with a third curvature. In view of Fig. 4, the second surface 42 of the plunger rod head comprises a third curvature of a smooth, convex nature, curving inwardly away from the tube 2. The convex curvature of the second surface 42 of the plunger rod head 4 allows the user to further support at least a part of at least one of their fingers and/or at least one of their thumbs upon for injection or aspiration.

In the embodiment shown in Fig. 4, the first non-slip surface parts 101 and the second non-slip surface parts 102 are connected by an intermediate non-slip surface part 112, forming a single, connected second non-slip surface layer on the plunger rod head 4. Alternatively, the first non-slip surface part 101 and the second non-slip surface part 102 can extend on one or more side surfaces of the plunger rod head 4. When the user places their finger(s) or thumb(s), or a combination thereof, on the edges of the plunger rod head 4, this can provide improved grip and comfort for the user, leading to a steadier, improved grasp of the syringe 1.

The present invention has been described with reference to a number of exemplary embodiments as shown in the drawings. Modifications and alternative implementations of some parts or elements are possible, and are included in the scope of protection as long as they fall under the subject-matter defined by the appended claims.

## Claims

1. A syringe (1) for injection and aspiration, comprising:
a tube (2) arranged to hold a liquid material;
a plunger rod (3) having a plunger (43) movably arranged within the tube (2), and a plunger rod head (4) attached to the plunger rod (3) opposite from the plunger (43), the plunger rod head (4) having a first surface (41) and a second surface (42) opposite to the first surface (41); and
a finger grip component (5), wherein
a first non-slip surface part (101) is provided on at least a part of the first surface (41) and a second non-slip surface part (102) is provided on at least a part of the second surface (42) of the plunger rod head (4);
the finger grip component (5) comprises a first portion (51) extending away from a longitudinal axis (A) of the syringe (1), a second portion (52) extending away from the longitudinal axis (A) of the syringe (1), **characterized in that** the finger grip component (5) further comprises a partial circumferential aperture (56);
the finger grip component (5) is a one-piece body attached to the tube (2), comprising
an injection finger support surface (61) with a third non-slip surface part (103) positioned in the first portion (51) and arched in a direction perpendicular to the longitudinal axis (A) with a first curvature wherein the injection finger support surface (61) and the third non-slip surface part (103) face away from the first surface (41) of the plunger rod head (4), and
an aspiration finger support surface (62) with a fourth non-slip surface part (104) positioned in the second portion (52) and arched in a direction perpendicular to the longitudinal axis (A) with a second curvature, wherein the aspiration finger support surface (62) and the fourth non-slip surface part (104) face toward the second surface (42) of the plunger rod head (4), wherein the second surface (42) faces the finger grip component (5), and
the third non-slip surface part (103) and the fourth non-slip surface part (104) are connected by an intermediate non-slip surface part (110), wherein the partial circumferential aperture (56) and the intermediate non-slip surface part (110) are on opposite circumferential sides.

2. The syringe according to claim 1, wherein the first, second, third and fourth non-slip surface parts comprise a first material which is softer than a second material of the associated first surface, second surface, first portion and second portion, respectively.

3. The syringe according to claim 2, wherein the first material is a thermoplastic elastomer.

4. The syringe according to claim 2 or 3, wherein the second material is a polypropylene material.

5. The syringe according to any one of claims 1-4, wherein the injection finger support surface (61) and/or the aspiration finger support surface (62) have a dimension perpendicular to the longitudinal axis (A) of at least 1 cm, e.g. at least 2 cm.

6. The syringe according to any one of claims 1-5, wherein a dimension perpendicular to the longitudinal axis (A) of the injection finger support surface (61) is larger than a dimension perpendicular to the longitudinal axis (A) of the aspiration finger support surface (62).

7. The syringe according to any one of claims 1-6, wherein the third non-slip surface part (103) and the fourth non-slip surface part (104) extend on one or more side surfaces of the finger grip component (5).

8. The syringe according to any one of claims 1-7, wherein the second surface (42) of the plunger rod head (4) is arched in a direction perpendicular to the longitudinal axis (A) with a third curvature.

9. The syringe according to any one of claims 1-8, wherein the first non-slip surface parts (101) and the second non-slip surface parts (102) are connected by an intermediate non-slip surface part (112).

10. The syringe according to any one of claims 1-6, wherein the first non-slip surface parts (101) and the second non-slip surface parts (102) extend on one or more side surfaces of the plunger rod head (4).

11. The syringe according to any one of claims 1-10, wherein the finger grip component (5) comprises an opening (55) aligned with the longitudinal axis (A).

12. The syringe according to any one of claims 1-11, wherein the finger grip component (5) has a partial circumferential aperture (56) parallel to the longitudinal axis (A).

## Patentansprüche

1. Spritze (1) zum Injizieren und Aspirieren, umfassend:
ein Rohr (2), das zum Aufnehmen eines flüssigen Materials ausgebildet ist;
eine Kolbenstange (3) mit einem Kolben (43), der beweglich innerhalb des Rohrs (2) angeordnet ist, und einem Kolbenstangenkopf (4), der an der Kolbenstange (3) gegenüber dem Kolben (43) angebracht ist, wobei der Kolbenstangenkopf (4) eine erste Oberfläche (41) und eine zweite Oberfläche (42) gegenüber der ersten Oberfläche (41) aufweist; und
einem Fingergriffelement (5), wobei ein erster rutschfester Oberflächenteil (101) an mindestens einem Teil der ersten Oberfläche (41) und ein zweiter rutschfester Oberflächenteil (102) an mindestens einem Teil der zweiten Oberfläche (42) des Kolbenstangenkopfes (4) vorgesehen ist;
wobei die Fingergriffkomponente (5) einen ersten Abschnitt (51), der sich von einer Längsachse (A) der Spritze (1) weg erstreckt, und einen zweiten Abschnitt (52) umfasst, der sich von der Längsachse (A) der Spritze (1) weg erstreckt,
**dadurch gekennzeichnet, dass** die Fingergriffkomponente (5) ferner eine teilweise umlaufende Öffnung (56) umfasst; und die Fingergriffkomponente (5) ein einteiliger Körper ist, der an dem Rohr (2) befestigt ist und eine Injektionsfingerauflagefläche (61) umfasst mit einem dritten rutschfesten Oberflächenteil (103), das in dem ersten Abschnitt (51) positioniert ist und in einer Richtung senkrecht zur Längsachse (A) mit einer ersten Krümmung gewölbt ist, wobei die Injektionsfingerauflagefläche die Fingergriffkomponente (5) ein einteiliger Körper ist, der an dem Rohr (2) befestigt ist und eine Injektionsfingerauflagefläche (61) mit einem dritten rutschfesten Oberflächenteil (103) umfasst, das in dem ersten Abschnitt (51) positioniert ist und in einer Richtung senkrecht zur Längsachse (A) mit einer ersten Krümmung gewölbt ist, wobei die Injektionsfingerauflagefläche (61) und das dritte rutschfeste Oberflächenteil (103) von der ersten Oberfläche (41) des Kolbenstangenkopfes (4) wegweisen, und eine Saugfingerauflagefläche (62) mit einem vierten rutschfesten Oberflächenteil (104) aufweist, der im zweiten Abschnitt (52) angeordnet ist und in einer Richtung senkrecht zur Längsachse (A) mit einer zweiten Krümmung gewölbt ist, wobei die Saugfinger-Stützfläche (62) und der vierte rutschfeste Oberflächenteil (104) zur zweiten Fläche (42) des Kolbenstangenkopfes (4) hin ausgerichtet sind, wobei die zweite Fläche (42) zur Fingergriffkomponente (5) zugewandt ist, und der dritte rutschfeste Oberflächenteil (103) und der vierte rutschfeste Oberflächenteil (104) durch einen mittleren rutschfesten Oberflächenteil (110) verbunden sind, wobei sich die teilweise umlaufende Öffnung (56) und der mittlere rutschfeste Oberflächenteil (110) auf gegenüberliegenden Umfangsseiten befinden.

2. Die Spritze gemäß Anspruch 1, wobei der erste, zweite, dritte und vierte rutschfeste Oberflächenteil ein erstes Material umfassen, das weicher ist als ein zweites Material der zugehörigen ersten Oberfläche, zweiten Oberfläche, ersten Abschnitts und zweiten Abschnitts.

3. Die Spritze gemäß Anspruch 2, wobei das erste Material ein thermoplastisches Elastomer ist.

4. Spritze nach Anspruch 2 oder 3, wobei das zweite Material ein Polypropylen Material ist.

5. Spritze nach einem der Ansprüche 1 bis 4, wobei die Injektionsfingerauflagefläche (61) und/oder die Aspirationsfingerauflagefläche (62) eine Abmessung senkrecht zur Längsachse (A) von mindestens 1 cm, beispielsweise mindestens 2 cm, aufweisen.

6. Spritze nach einem der Ansprüche 1 bis 5, wobei eine Abmessung senkrecht zur Längsachse (A) der Injektionsfingerauflagefläche (61) größer ist als eine Abmessung senkrecht zur Längsachse (A) der Aspirationsfingerauflagefläche (62).

7. Spritze nach einem der Ansprüche 1 bis 6, wobei sich der dritte rutschfeste Oberflächenteil (103) und der vierte rutschfeste Oberflächenteil (104) auf einer oder mehreren Seitenflächen des Fingergriffelements (5 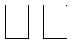 ) erstrecken.

8. Die Spritze gemäß einem der Ansprüche 1 bis 7, wobei die zweite Fläche (42) des Kolbenstangenkopfes (4) in einer Richtung senkrecht zur Längsachse (A) mit einer dritten Krümmung gewölbt ist.

9. Die Spritze gemäß einem der Ansprüche 1 bis 8, wobei die ersten rutschfesten Oberflächenteile (101) und die zweiten rutschfesten Oberflächenteile (102) durch einen dazwischenliegenden rutschfesten Oberflächenteil (112) verbunden sind.

10. Die Spritze gemäß einem der Ansprüche 1 bis 6, wobei sich die ersten rutschfesten Oberflächenteile (101) und die zweiten rutschfesten Oberflächenteile (102) über eine oder mehrere Seitenflächen des Kolbenstangenkopfes (4) erstrecken.

11. Die Spritze gemäß einem der Ansprüche 1 bis 10, wobei die Fingergriffkomponente (5) eine mit der Längsachse (A) ausgerichtete Öffnung (55) umfasst.

12. Die Spritze gemäß einem der Ansprüche 1 bis 11, wobei die Fingergriffkomponente (5) eine teilweise umlaufende Öffnung (56) parallel zur Längsachse (A) aufweist.

## Revendications

1. Seringue (1) pour injection et aspiration, comprenant :
un tube (2) agencé pour contenir un matériau liquide ;
une tige de piston (3) comportant un piston (43) disposé de manière mobile à l'intérieur du tube (2), et une tête de tige de piston (4) fixée à la tige de piston (3) à l'opposé du piston (43), la tête de tige de piston (4) présentant une première surface (41) et une deuxième surface (42) opposée à la première surface (41) ; et
un composant de préhension pour les doigts (5),
dans laquelle
une première partie de surface antidérapante (101) est prévue sur au moins une partie de la première surface (41), et une deuxième partie de surface antidérapante (102) est prévue sur au moins une partie de la deuxième surface (42) de la tête de tige de piston (4) ;
le composant de préhension pour les doigts (5) comprend une première portion (51) s'étendant en éloignement d'un axe longitudinal (A) de la seringue (1) et une deuxième portion (52) s'étendant en éloignement de l'axe longitudinal (A) de la seringue (1),
**caractérisée en ce que**
le composant de préhension pour les doigts (5) présente en outre une ouverture circonférentielle partielle (56) ;
le composant de préhension pour les doigts (5) est un corps monobloc fixé au tube (2), comprenant
une surface d'injection et d'appui pour les doigts (61) munie d'une troisième partie de surface antidérapante (103) positionnée dans la première portion (51) et arquée dans une direction perpendiculaire à l'axe longitudinal (A) avec une première courbure, la surface d'injection et d'appui pour les doigts (61) et la troisième partie de surface antidérapante (103) étant détournées de la première surface (41) de la tête de tige de piston (4), et
une surface d'aspiration et d'appui pour les doigts (62) munie d'une quatrième partie de surface antidérapante (104) positionnée dans la deuxième portion (52) et arquée dans une direction perpendiculaire à l'axe longitudinal (A) avec une deuxième courbure, la surface d'aspiration et d'appui pour les doigts (62) et la quatrième partie de surface antidérapante (104) étant tournées vers la deuxième surface (42) de la tête de tige de piston (4), la deuxième surface (42) étant tournée vers le composant de préhension pour les doigts (5), et
la troisième partie de surface antidérapante (103) et la quatrième partie de surface antidérapante (104) étant reliées par une partie de surface antidérapante intermédiaire (110), l'ouverture circonférentielle partielle (56) et la partie de surface antidérapante intermédiaire (110) étant situées sur des côtés circonférentiels opposés.

2. Seringue selon la revendication 1,
dans laquelle les première, deuxième, troisième et quatrième parties de surface antidérapante comprennent un premier matériau qui est plus souple qu'un deuxième matériau de la première surface, de la deuxième surface, de la première portion et de la deuxième portion associées, respectivement.

3. Seringue selon la revendication 2,
dans laquelle le premier matériau est un élastomère thermoplastique.

4. Seringue selon la revendication 2 ou 3,
dans laquelle le deuxième matériau est un matériau en polypropylène.

5. Seringue selon l'une des revendications 1 à 4,
dans laquelle la surface d'injection et d'appui pour les doigts (61) et/ou la surface d'aspiration et d'appui pour les doigts (62) ont une dimension perpendiculaire à l'axe longitudinal (A) d'au moins 1 cm, par exemple d'au moins 2 cm.

6. Seringue selon l'une des revendications 1 à 5,
dans laquelle une dimension perpendiculaire à l'axe longitudinal (A) de la surface d'injection et d'appui pour les doigts (61) est supérieure à une dimension perpendiculaire à l'axe longitudinal (A) de la surface d'aspiration et d'appui pour les doigts (62).

7. Seringue selon l'une des revendications 1 à 6,
dans laquelle la troisième partie de surface antidérapante (103) et la quatrième partie de surface antidérapante (104) s'étendent sur une ou plusieurs surfaces latérales du composant de préhension pour les doigts (5).

8. Seringue selon l'une des revendications 1 à 7,
dans laquelle la deuxième surface (42) de la tête de tige de piston (4) est arquée dans une direction perpendiculaire à l'axe longitudinal (A) avec une troisième courbure.

9. Seringue selon l'une des revendications 1 à 8,
dans laquelle les premières parties de surface antidérapante (101) et les deuxièmes parties de surface antidérapante (102) sont reliées par une partie de surface antidérapante intermédiaire (112).

10. Seringue selon l'une des revendications 1 à 6,
dans laquelle les premières parties de surface antidérapante (101) et les deuxièmes parties de surface antidérapante (102) s'étendent sur une ou plusieurs surfaces latérales de la tête de tige de piston (4).

11. Seringue selon l'une des revendications 1 à 10,
dans laquelle le composant de préhension pour les doigts (5) présente un orifice (55) aligné avec l'axe longitudinal (A).

12. Seringue selon l'une des revendications 1 à 11,
dans laquelle le composant de préhension pour les doigts (5) présente une ouverture circonférentielle partielle (56) parallèle à l'axe longitudinal (A).
